(19) Europäisches Patentamt
European Patent Office
Office européen des brevets

(11) **EP 2 540 334 B1**

(12) **EUROPÄISCHE PATENTSCHRIFT**

(45) Veröffentlichungstag und Bekanntmachung des Hinweises auf die Patenterteilung:
**15.10.2014 Patentblatt 2014/42**

(51) Int Cl.:
***A61M 16/08*** *(2006.01)*     ***A61M 16/10*** *(2006.01)*
***A61M 16/22*** *(2006.01)*     ***A61M 16/20*** *(2006.01)*

(21) Anmeldenummer: **11172088.4**

(22) Anmeldetag: **30.06.2011**

(54) **Beatmungssystem**

Breathing system

Système de respiration

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**02.01.2013 Patentblatt 2013/01**

(73) Patentinhaber: **Dräger Medical GmbH**
**23558 Lübeck (DE)**

(72) Erfinder:
• **Heesch, Ralf**
**23568 Lübeck (DE)**
• **Schnaars, Henryk**
**23552 Lübeck (DE)**

(74) Vertreter: **UEXKÜLL & STOLBERG**
**Patentanwälte**
**Beselerstrasse 4**
**22607 Hamburg (DE)**

(56) Entgegenhaltungen:
**EP-A1- 0 911 054     DE-B3-102004 052 398**
**DE-C1- 10 041 007     US-A- 4 207 884**
**US-A- 5 033 464**

**Beschreibung**

Die vorliegende Erfindung betrifft ein Beatmungssystem zum Zuführen eines Narkosegases mit einem Y-Stück, das zur Verbindung mit einem Patienten vorgesehen ist, mit einem Beatmungskreis, in dem ein inspiratorischer Zweig und ein exspiratorischer Zweig vorgesehen sind, die sich von dem Y-Stück weg erstrecken, wobei in dem exspiratorischen Zweig eine Verzweigung vorgesehen ist, wobei von der Verzweigung eine erste Zuleitung zu einem Narkosegasfortleitungsventil führt und wobei von der Verzweigung eine zweite Zuleitung zu einem Reservoir führt, wobei von der Verzweigung eine zweite Zuleitung zu einem Reservoir führt, die eine Querschnittsfläche $A_{zuleitung2}$ und eine Länge $l_{zuleitung2}$ aufweist, und wobei das Narkosegasfortleitungsventil einen Ventilkörper mit einer Masse $m_{ventil}$ aufweist.

Derartige, aus dem Stand der Technik wie beispielsweise der EP 0 894 506 B1 bekannte Beatmungssysteme haben zunächst den Vorteil, dass das Narkosegas in einem Beatmungskreis geführt ist, sodass ein großer Teil des kostspieligen Narkosegases wiederverwendet werden kann. Lediglich $CO_2$ muss mit Hilfe eines Absorbers aus dem umlaufenden Gasstrom entfernt werden, und die Sauerstoffkonzentration muss auf einem vorgegebenen Niveau gehalten werden.

Mit Hilfe des ebenfalls in dem Kreis enthaltenen Handbeatmungsbeutels ist es für einen Anästhesist möglich, den beim Einatmen wirkenden Druck und die Dauer des Einatmens von Hand einzustellen.

Aus der DE 10 2004 052 398 B3 , von der die vorliegende Erfindung ausgeht, ist ebenfalls ein Beatmungssystem bekannt, das ein Y-Stück und einen damit verbundenen Beatmungskreis mit einem exspiratorischen und einem inspriratotrischen Zweig umfasst. Außerdem sind im expiratorischen Zweig eine Verzweigung und sich daran anschließend eine erste Zuleitung zu einem Narkosegasfortleitungsventil und eine zweite Zuleitung zu einem Reservoir vorgesehen.

Die U.S. 4,207,884 beschreibt ein Ventil für ein Beatmungssystem.

Schließlich ist aus der DE 100 41 007 C1 ebenfalls ein Beatmungssystem mit einem inspiratorischen und einem exspiratorischen Zweig bekannt, wobei im exspiratorischen Zweig eine Verzweigung zu einem Narkosegasfortleitungsventil vorgesehen ist.

Allerdings ergibt sich bei dem aus der EP 0 894 506 B1 bekannten System folgendes Problem. Beim Ausatmen soll der Widerstand bzw. der dem Ausatmen entgegenwirkende Druck einen bestimmten Wert nicht übersteigen, wobei insbesondere am Ende des Ausatem-Zyklus der Druck im exspiratorischen Zweig anwachsen kann, beispielsweise wenn die Kapazität des Reservoirs nicht ausreichend ist. Damit dieser Widerstand nicht unzulässig hoch wird, ist im exspiratorischen Zweig ein sogenanntes Narkosegasfortleitungsventil (im Folgenden "NGF-Ventil") vorgesehen, das bei einem Überdruck im exspiratorischen Zweig von 1,2 $\pm$ 0,1 mbar öffnet. Dieses Ventil ist im Stand der Technik in der Regel so aufgebaut, dass ein Ventilkörper mittels einer Feder gegen einen Ventilsitz gedrückt wird, wobei die Vorspannung der Feder die Schwelle bestimmt, bei der das Ventil öffnet.

Die Strömung im exspiratorischen Zweig, insbesondere am Beginn des Ausatmens, ist nicht konstant und auch der Druck, der sich beim Ausatmen am NGF-Ventil aufbaut, schwankt und hat kurzzeitige Spitzen bzw. große Druckgradienten im Bereich von $\frac{\Delta p}{\Delta t} = \frac{5 mbar}{0,5 s}$ , sodass sich an dem NGF-Ventil kurzzeitig über der zuvor erwähnten Schwelle liegende Drücke aufbauen, die dadurch zustande kommen, dass das Gas in der Zuleitung zum Handbeatmungsbeutel eine Trägheit hat. Dadurch kommt es dazu, dass das NGF-Ventil ebenfalls kurzzeitig öffnet, obwohl sich im exspiratorischen Zweig nicht für einen längeren Zeitraum ein Druck oberhalb der Schwelle aufgebaut hat. Es kann also zu einem sogenannten "Klappern" des NGF-Ventils kommen, was zu einem unnötigen Verlust von Narkosegas führt.

Ausgehend von dem zuvor beschriebenen Stand der Technik ist es daher die Aufgabe der vorliegenden Erfindung, ein Beatmungssystem bereitzustellen, bei dem sich im exspiratorischen Zweig kein zu hoher, dem Ausatmen entgegenwirkender Druck aufbauen kann und das dennoch die Verluste an Narkosegas minimal hält.

Diese Aufgabe wird gelöst durch ein Beatmungssystem zum Zuführen eines Narkosegases mit einer Dichte $\rho_{Gas}$, mit einem Y-Stück, das zur Verbindung mit einem Patienten vorgesehen ist, mit einem Beatmungskreis, in dem ein inspiratorischer Zweig und ein exspriatorischer Zweig vorgesehen sind, die sich von dem Y-Stück weg erstrecken, wobei in dem exspiratorischen Zweig eine Verzweigung vorgesehen ist, wobei von der Verzweigung eine erste Zuleitung zu einem NGF-Ventil führt, wobei von der Verzweigung eine zweite Zuleitung zu einem Reservoir führt, die eine mittlere Querschnittsfläche $A_{Zuleitung2}$ und eine Länge $l_{Zuleitung2}$ aufweist, wobei das NGF-Ventil einen Ventilkörper mit einer Masse $m_{Ventil}$ aufweist, der durch Schwerkraftwirkung hin zu der ersten Zuleitung gegen einen kreisringförmigen Ventilsitz mit einem Durchmesser $d_{Ventil}$ gedrückt wird, und wobei ein Vorspannmittel vorgesehen ist, das auf den Ventilkörper eine Vorspannkraft entgegen der Wirkung der Schwerkraft ausübt, sodass die Masse $m_{Ventil}$ und die Vorspannkraft einen Schwellendruck $\Delta P_{Schwelle}$ festlegen, um den der Druck in der ersten Zuleitung über dem auf der der ersten Zuleitung abgewandten Seite des Narkosegasfortleitungsventils mindestens liegen muss, damit dieses öffnet.

Bei herkömmlichen, aus dem Stand der Technik bekannten NGF-Ventilen wird der Ventilkörper mit Hilfe eines Vorspannmittels wie einer Feder gegen den Ventilsitz gedrückt, wobei die Federkraft und die in die gleiche Richtung wirkende Gewichtskraft des Ventilkörpers zusammen den Schwellendruck bestimmen, bei der das Ventil öffnet. Da der Schwellendruck jedoch sehr gering sein muss, dürfen auch die Masse und die Federkraft nicht sehr groß sein.

Bei der erfindungsgemäßen Lösung wird durch das Vorspannmittel, das entgegen der Richtung der Schwerkraft wirkt, ein Teil der Schwerkraft kompensiert, die auf den Ventilkörper wirkt und aufgrund derer das Ventil schließt. Dies ermöglicht, dass der Ventilkörper eine deutlich größere Masse haben kann und damit träger sein kann, sodass lediglich kurzzeitige Druckspitzen bzw. besser hohe Druckgradienten von etwa $\frac{\Delta p}{\Delta t} = \frac{5mbar}{0,5s}$ und damit eine lediglich kurzzeitige Kraftwirkung auf den Ventilkörper nicht ausreichen, den Ventilkörper von dem Ventilsitz abzuheben. Die Masse des Ventilkörpers kann daher an die übrigen Teile des Beatmungssystems sowie die Dichte $\rho_{Gas}$ des verwendeten Narkosegases angepasst werden, um die aufgrund des Aufbaus des Beatmungssystems notwendige Trägheit des Ventils zu erreichen.

Insbesondere erfolgt die Anpassung in Abhängigkeit von der Länge der zweiten Zuleitung zu dem Reservoir und des Querschnitts dieser Zuleitung sowie des treibenden Drucks des Patienten, so dass der in der Zuleitung zum Reservoir auftretende Strömungswiderstand und die daraus resultierenden Druckgradienten am NGF-Ventil nicht ausreichen, dessen Ventilkörper aus seiner geschlossenen Stellung zu bewegen, in der er an dem Ventilsitz anliegt. Dadurch öffnet das NGF-Ventil nur dann, wenn sich im exspiratorischen Zweig für einen längeren Zeitraum ein Druck aufbaut, dessen Differenz zu dem Umgebungsdruck über dem Schwellendruck $\Delta P_{Schwelle}$ liegt, sodass der Verlust an Narkosegas minimiert wird.

In bevorzugter Weise ist die Masse $m_{Ventil}$ des Ventilkörpers derart gewählt, dass

$$m_{Ventil} > \frac{\sqrt{2} \cdot \pi^2 \cdot l_{Zuleitung\,2}^2 \cdot \dot{V}_{Schnitt} \cdot \rho_{Gas}^2 \cdot d_{Ventil}^3 \cdot (\Delta P - \Delta P_{Schwelle})}{8 \cdot A_{Zuleitung\,2}^2 \cdot \Delta P^2 \cdot \sqrt{\frac{\rho_{Gas}}{\Delta P}}} \qquad (1)$$

gilt, wobei der Parameter $\dot{V}_{Schnitt}$ entsprechend $\dot{V}_{Schnitt} \geq 10 l/min$ gewählt ist und für den treibenden Druck $\Delta P$ beim Ausatmen des Patienten $\Delta P = 5mbar$ angenommen wird. Für dem Schwellendruck wird $\Delta P_{Schwelle} = 1,1$ mbar gewählt, und die Dichte $\rho_{Gas}$ des Narkosegases liegt zwischen 1,12 kg/m$^3$ und 2,19 kg/m$^3$. Hierbei wird eine Atemgastemperatur von 20°C zugrunde gelegt. Wenn die Dichte des Atemgases festliegt, das in dem System verwendet werden soll, kann der entsprechende Wert für die Dichte in Gleichung (1) eingesetzt werden, um so das System entsprechend anzupassen, wobei dieser Dichtewert aber in dem vorher definierten Intervall liegt.

Damit ist durch die Gleichung (1) eine Vorschrift zu Dimensionierung des Ventilkörpers in Abhängigkeit von den weiteren relevanten Abmessungen innerhalb des Beatmungssystems gegeben. Wenn diese Vorschrift eingehalten wird, wird zuverlässig sichergestellt, dass das NGF-Ventil aufgrund der hinreichenden Trägheit des Ventilkörpers nicht bereits bei kurzzeitigen hohen Druckgradienten im Bereich von $\frac{\Delta p}{\Delta t} = \frac{5mbar}{0,5s}$ öffnet, die aus dem Strömungswiderstand in der zweiten Zuleitung zum Reservoir bzw. Handbeatmungsbeutel und der Trägheit des darin vorhandenen Gases resultieren. Vielmehr öffnet das NGF-Ventil erst dann, wenn der Druck im exspiratorischen Zweig für eine längere Zeit um den Schwellendruck über dem Umgebungsdruck liegt.

Derartige Druckgradienten treten zyklisch während des Beatmungsbetriebes durch den fortwährenden Wechsel zwischen Einatmung und Ausatmung des Patienten auf, wobei die Trägheit des Gases in der Zuleitung zum Reservoir dazu führt, dass sich die Druckgradienten gerade in der Zuleitung zum NGF-Ventil aufbauen. Die Atemfrequenz und damit die Häufigkeit solcher Spitzen liegt bei einem typischen Patienten im Bereich von 6 bis 15 Atemhüben pro Minute.

Der Gleichung (1) liegen die folgenden Randbedingungen und Überlegungen zugrunde.

Es wird davon ausgegangen, dass beim Ausatmen aufgrund des treibenden Drucks $\Delta P$ des Patienten Narkosegas entlang des exspiratorischen Zweigs strömt, und zwar sowohl entlang der ersten Zuleitung zu dem NGF-Ventil als auch entlang der zweiten Zuleitung zu dem Reservoir bzw. dem Handbeatmungsbeutel. Für die Trägheit $I_{pneu}$ des Gases in der zweiten Zuleitung gilt

$$I_{pneu} = \frac{\Delta P}{\left(\dfrac{d}{dt}\dot{V}\right)} = \frac{\rho_{gas} \cdot l_{Zuleitung\,2}}{A_{Zuleitung\,2}} \qquad (2),$$

wobei von einer Zuleitung mit im Wesentlichen kreisförmigem Querschnitt ausgegangen wird. Außerdem gilt für die Trägheit des NGF-Ventils

$$I_{NGF} = \frac{2 \cdot m_{Ventilkörper}}{\pi^2 \cdot d_{Ventil}^3} \cdot \sqrt{\frac{2 \cdot \rho_{Gas}}{\Delta P}} \qquad (3),$$

wobei ein kreisförmiger Ventilsitz mit einem Durchmessen $d_{ventil}$ angenommen wird. Der Ventilsitz ist derjenige Flächenbereich, an dem der Ventilkörper an dem Ventilgehäuse anliegt. Damit kann der Volumenstrom als Funktion der Zeit t durch die zweite Zuleitung zu dem Reservoir über die Beziehung

$$\dot{V}_{Zuleitung\,2} = \frac{t \cdot \Delta P}{I_{pneu}} \qquad (4)$$

berechnet werden, während für den zeitlichen Verlauf des Volumenstroms durch das NGF-Ventil

$$\dot{V}_{NGF} = \frac{1}{2} \cdot t^2 \frac{\Delta P - \Delta P_{Schwelle}}{I_{pneu}} \qquad (5)$$

gilt. Das bedeutet, dass der Volumenstrom $\dot{V}_{Zuleitung\,2}$ durch die zweite Zuleitung zum Reservoir bzw. Handbeatmungsbeutel linear mit der Zeit zunimmt, während der Volumenstrom $\dot{V}_{NGF}$ durch das NGF-Ventil einen quadratischen Verlauf hat.

Dies ergibt sich dadurch, dass der Volumenstrom $\dot{V}_{Zuleitung\,2}$ in das Reservoir bei konstantem treibendem Druck $\Delta P$ proportional zur Zeit t ist, während der Volumenstrom $\dot{V}_{NGF}$ durch das NGF-Ventil proportional zu der Größe des Spalts zwischen Ventilkörper und Ventilsitz ist. Dieser wiederum wird durch die Bewegung des Ventilkörpers vergrößert, wobei die Beschleunigung des Ventilkörpers proportional zur Kraft ist, die auf ihn ausgeübt wird. Diese ist wiederum proportional zum treibenden Druck $\Delta P$. Wenn dieser konstant ist, gibt es eine konstante Beschleunigung und die Größe des Spalts ist proportional zum Quadrat der Zeit $t^2$.

Nun wird für die Dimensionierung des Beatmungssystems gefordert, dass der Volumenstrom $\dot{V}_{NCF}$ durch das NGF-Ventil den Volumenstrom durch die Zuleitung erst nach einer relativ großen Zeit übersteigen soll und nicht bereits bei den zuvor definierten hohen Druckgradienten. Das NGF-Ventil soll also nur sehr langsam öffnen, was bedeutet, dass der Schnittpunkt $\dot{V}_{Schnitt}$ zwischen beiden Volumenströmen $\dot{V}_{Zuleitung\,2}$, $\dot{V}_{NGF}$ erst bei einem Zeitpunkt $t_{Schnitt}$, der größer als die Dauer der auftretenden Druckgradienten ist, bzw. bei einem relativen hohen Volumenstrom liegen muss.

Wenn man zur Ermittlung des Schnittpunkts $\dot{V}_{Schnitt}$ die Gleichungen (4) und (5) gleich setzt und dabei für die Trägheiten die Gleichungen (2) und (3) verwendet, ergibt sich zunächst eine Beziehung für $t_{Schnitt}$. Damit lässt sich dann durch Einsetzen der Schnittzeitpunkts $t_{Schnitt}$ in Gleichung (4) zusammen mit Gleichung (2) die Beziehung

$$\dot{V}_{Schnitt} = \frac{4 \cdot A_{Zuleitung\,2} \cdot m_{Ventil} \cdot \Delta P^2 \sqrt{\frac{2 \cdot \rho_{Gas}}{\Delta P}}}{\pi^2 \cdot l^2_{Zuleitung\,2} \cdot \rho^2_{Gas} \cdot d^3_{Ventil} \cdot (\Delta P - \Delta P_{Schwelle})} \qquad (6)$$

aufstellen.

Hieraus wiederum ergibt sich durch Umstellen die Bedingung für die Masse des Ventilkörpers $m_{Ventil}$ gemäß Gleichung (1) . Dabei sollte für $\dot{V}_{Schnitt}$ die Beziehung $\dot{V}_{Schnitt} \geq 10l/min$, vorzugweise $\dot{V}_{Schnitt} \geq 60l/min$ gelten.

Wenn $\dot{V}_{Schnitt} \geq 10l/min$ gewählt wird, ergibt sich insbesondere im Bereich der Pädiatrie schon ein signifikanter Effekt. Ein Wert von $\dot{V}_{Schnitt} \geq 60l/min$ ist auf einen Erwachsenen abgestimmt und entspricht in etwa dem Spitzenvolumenstrom, den ein Erwachsener erreichen kann.

Während zuvor von kreisförmigen Querschnitten der Zuleitungen ausgegangen wurde, ist es klar, dass Abwandlungen mit nichtkreisförmigen Querschnitten von der Erfindung mit umfasst sind.

In einer bevorzugten Ausführungsform ist das NGF-Ventil in der Weise aufgebaut, dass es ein Ventilgehäuse aufweist, in dem der Ventilsitz vorgesehen ist, wobei der Ventilsitz in einer senkrecht zur Richtung der Schwerkraft verlaufenden Sitzebene verläuft und in eine der Richtung der Schwerkraft entgegengesetzte Richtung weist und wobei der Ventilkörper derart angeordnet ist, dass er unter Wirkung der Schwerkraft auf dem Ventilsitz aufliegt.

Dabei ist es weiter bevorzugt, wenn als Vorspannmittel eine Schraubenfeder vorgesehen ist, die mit einem ersten Ende an der dem Ventilkörper anliegt, die entgegen der Wirkung der Schwerkraft auf den Ventilkörper drückt und die auf der in Richtung der Schwerkraft weisenden Seite des Ventilkörpers angeordnet ist.

Um die Vorspannung durch die Feder und damit den Schwellendruck $\Delta P$ in einfacher Weise nachjustieren zu können, ist es ferner bevorzugt, dass eine Stellschraube im Ventilgehäuse axial verstellbar gehaltert ist, wobei sich das zweite Ende der Schraubenfeder an der Stellschraube abstützt.

Die vorliegende Erfindung wird im Folgenden anhand einer lediglich ein bevorzugtes Ausführungsbeispiel darstellenden Zeichnung erläutert, wobei

Fig. 1     eine schematische Darstellung des Ausführungsbeispiels eines erfindungsgemäßen Beatmungssystems zeigt und

Fig. 2     eine Schnittdarstellung des in dem Beatmungssystem aus Fig. 1 verwendeten NGF-Ventils ist.

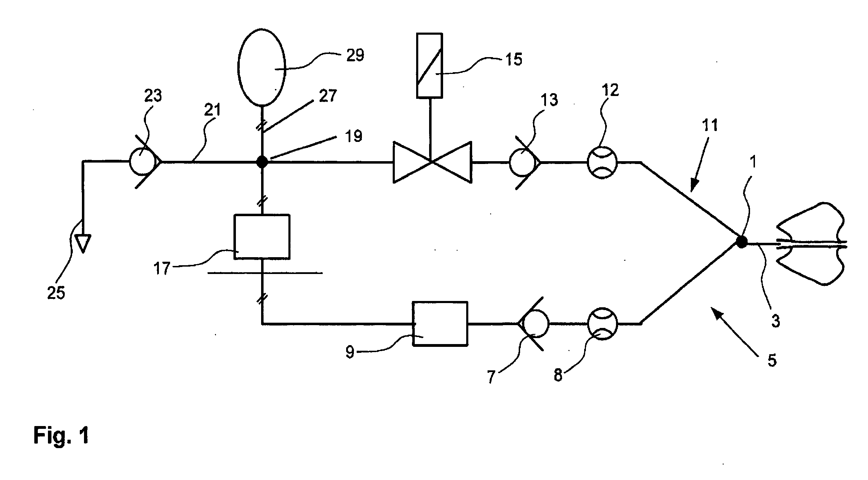

Das in Figur 1 dargestellte Ausführungsbeispiel eines Beatmungssystems umfasst einen hier nur schematisch dargestellten Beatmungskreis mit einem Y-Stück 1, von dem eine Anschlussleitung 3 ausgeht, über die ein Patient an das Beatmungssystem angeschlossen werden kann. Der Beatmungskreis umfasst ferner von dem Y-Stück 1 ausgehend einen inspiratorischen Zweig 5, in dem ein erstes Rückschlagventil 7, eine erste Messeinrichtung 8 zum Messen des Volumenstroms im inspiratorischen Zweig 5 sowie eine Ventilations- und Gasdosiereinrichtung 9 vorgesehen sind.

Das Rückschlagventil 7 ist dabei so aufgebaut, dass in dem inspiratorischen Zweig 5 nur eine Strömung von der Ventilations- und Gasdosiereinrichtung 9 hin zu dem Y-Stück 1 stattfinden kann, während bei einer in umgekehrter Richtung verlaufenden Strömung das erste Rückschlagventil 7 schließt. In der Ventilations- und Gasdosiereinrichtung 9 ist ein Beatmungsantrieb, mit dem eine Strömung zum Y-Stück 1 und damit zum Patienten hin bewirkt werden kann, sowie eine Frischgasdosiereinrichtung vorhanden.

Außerdem geht von dem Y-Stück 1 ein exspiratorischer Zweig 11 aus, in dem eine zweite Messeinrichtung 12, ein zweites Rückschlagventil 13 sowie ein PEEP-Ventil 15 vorgesehen sind. Das zweite Rückschlagventil 13 ist derart angeordnet, dass eine Strömung vom Y-Stück 1 hin zum PEEP-Ventil 15 ermöglicht wird, während das zweite Rückschlagventil 13 bei einer Strömung in umgekehrter Richtung schließt.

Die Funktionsweise des PEEP-Ventils 15 (PEEP entspricht "positive endexspiratory pressure") ist derart, dass das PEEP-Ventil 15 während der Einatemphase geschlossen ist, sodass Gas, dass vom der Ventilations- und Gasdosiereinrichtung 9 eigentlich dem Patienten zugeführt werden soll, nicht über den exspiratorischen Zweig 11 wegströmen kann. In der Ausatemphase ist das PEEP-Ventil 15 derart eingestellt, dass es in dem Abschnitt zwischen dem PEEP-Ventil 15 und dem Y-Stück 1 einen positiven Druck aufrechterhält.

Des Weiteren weist der exspiratorische Zweig 11 einen $CO_2$-Absorber 17 auf, wobei in Richtung von dem Y-Stück 1 aus gesehen hinter dem $CO_2$-Absorber 17 der exspiratorische Zweig 11 und der inspiratorische Zweig 5 miteinander verbunden sind.

Außerdem ist in dem exspiratorischen Zweig 11 eine Verzweigung 19 vorgesehen, von der eine erste Zuleitung 21 ausgeht, an deren Ende ein Narkosegasfortleitungsventil (im Folgenden "NGF-Ventil") 23 angeordnet ist. Die Ausgangsseite des NGF-Ventils 23 ist wiederum mit einem nicht näher dargestellten Narkosegasfortleitungssystem 25 verbunden.

Darüber hinaus geht von der Verzweigung 19 eine zweite Zuleitung 27 aus, die eine vergleichsweise große Länge $l_{Zuleitung2}$ von mehr als 1 m aufweisen kann und sich zu einem Handbeatmungsbeutel 29, der im vorliegenden Ausführungsbeispiel das Reservoir bildet, erstreckt. Durch diese große Länge $l_{Zuleitung2}$ kann das Gas darin auch eine erhebliche Trägheit haben. Die zweite Zuleitung 27 weist eine Querschnittsfläche $A_{Zuleitung2}$ auf. In dem Fall, dass der Querschnitt der zweiten Zuleitung 27 über deren Länge variiert, müssten die Trägheiten des Atemgases in Abschnitten mit jeweils konstantem Querschnitt separat gemäß Gleichung (2) (s.o.) berechnet werden, wobei sich dann eine von Gleichung (1) abweichende Beziehung ergeben würde. Hier ist es aber für den Fachmann offensichtlich, wie die ensprechend umgestellte Gleichung aussehen muss.

Im Hinblick auf die Verzweigung 19 ist anzumerken, dass im vorliegenden Ausführungsbeispiel beide Zuleitungen 21, 27 von einem Punkt im exspiratorischen Zweig 11 ausgehen. Es ist aber auch denkbar, dass die Verzweigung aufgespalten ist, sodass der erste und der zweite Zuleitung 21, 27 beabstandet voneinander von dem exspiratorischen Zweig 11 ausgehen.

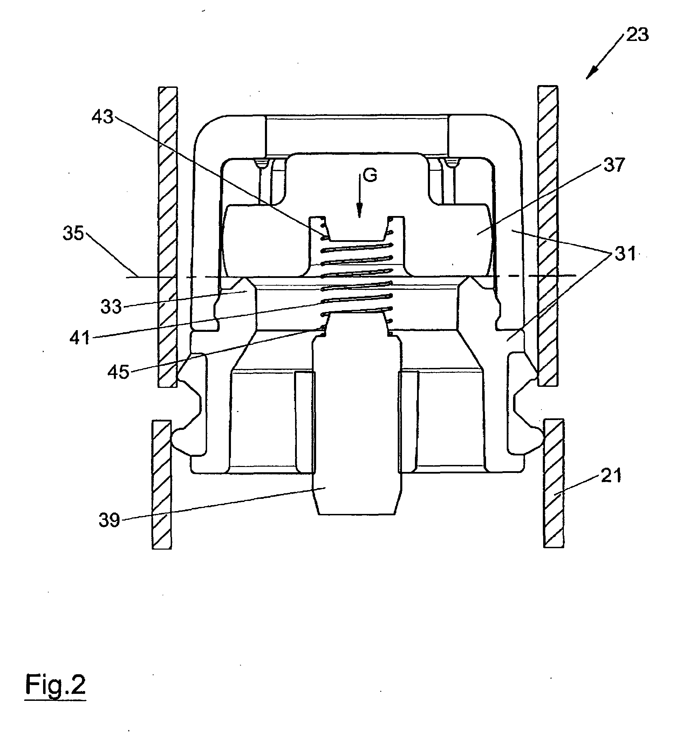

In Figur 2 ist das NGF-Ventil 23 im Detail dargestellt, und es ist zu erkennen, dass dieses ein Gehäuse 31 mit einem darin angeordneten kreisringförmigen Ventilsitz 33 aufweist, der sich in einer Sitzebene 35 erstreckt, die senkrecht zur Richtung der Schwerkraft G verläuft, und einen Durchmesser $d_{Ventil}$ hat. Außerdem ist in dem Gehäuse 31 ein beweglicher Ventilkörper 37 vorgesehen, der eine Masse $m_{Ventil}$ hat und aufgrund seines Gewichts an dem Ventilsitz 33 anliegt und das Ventil 23 somit verschließt. Schließlich ist in dem Gehäuse 31 eine Stellschraube 39 angeordnet, wobei zwischen dem sich im Gehäuseinneren befindlichen Ende der Stellschraube 39 und dem Ventilkörper 37 eine Schraubenfeder 41 angeordnet ist. Das erste Ende 43 der Schraubenfeder stützt sich dabei an dem Ventilkörper 37 ab, und das zweite Ende 45 liegt an der Stellschraube 39 an. Durch die Schraubenfeder 41 wird somit ein Teil der Gewichtskraft des Ventilkörpers 37 kompensiert, so dass die Gewichtskraft des Ventilkörpers 37 einerseits und die Vorspannung der Schraubenfeder 41 andererseits festlegen, bei welchem Schwellendruck $\Delta P_{Schwelle}$ das NGF-Ventil 23 öffnet, sofern dieser Schwellendruck $\Delta P_{Schwelle}$ durch die Druckdifferenz zwischen der Seite des NGF-Ventils 23, die der Zuleitung 21 zugewandt ist, und der Seite, die zum Narkosegasfortleitungssystem 25 weist, überschritten ist. Dabei kann durch Verstellen der Stellschraube 39 dieser Schwellendruck $\Delta P_{Schwelle}$ nachjustiert werden.

Schließlich ist in diesem zuvor beschriebenen Ausführungsbeispiel die Masse $m_{Ventil}$ des Ventilkörpers 37 gemäß der zuvor bereits erläuterten Gleichung

$$m_{Ventil} > \frac{\sqrt{2} \cdot \pi^2 \cdot l^2_{Zuleitung\,2} \cdot \dot{V}_{Schnitt} \cdot \rho^2_{Gas} \cdot d^3_{Ventil} \cdot (\Delta P - \Delta P_{Schwelle})}{8 \cdot A^2_{Zuleitung\,2} \cdot \Delta P^2 \cdot \sqrt{\dfrac{\rho_{Gas}}{\Delta P}}} \qquad (1)$$

festgelegt, um das NGF-Ventil 23 hinreichend träge auszugestalten, so dass nicht bereits kurzzeitige hohe Druckgradienten n ein Öffnen des NGF-Ventils 23 bewirken.

Die in dieser Gleichung enthaltenen Parameter beschreiben zum einen die Abmessungen von Teilen des Beatmungssystems. So gehen in die Gleichung (1) die Länge der zweiten Zuleitung 27 $l_{Zuleitung2}$, der Durchmesser des Ventilsitzes 31 $d_{Ventil}$, der am NGF-Ventil 23 eingestellte Schwellendruck $\Delta P_{Schwelle}$ (gewöhnlich etwa 1, 1 mbar) und die Querschnittsfläche $A_{Zuleitung2}$ der zweiten Zuleitung 27 ein. Wenn die Querschnittsform der zweiten Zuleitung entlang deren Länge variiert, muss Gleichung (1), wie bereits erläutert, in naheliegender Weise entsprechend umgestellt werden.

Zum anderen gehen weitere Parameter ein, die die beatmeten Patienten bzw. das verwendete Atemgas betreffen und ebenfalls ohne Weiteres bekannt sind. Hierbei handelt es sich um die Dichte des Atemgases $\rho_{Gas}$, die zwischen 1,12 Kg/m$^3$ und 2, 19 Kg/m$^3$ liegen kann, sodass hier ein vergleichsweise enger Bereich vorgegeben ist, um den treibenden Druck $\Delta P$, der durch den ausatmenden Patienten erzeugt wird und der bei 5 mbar liegt, und um den Schnittvolumenstrom $\dot{V}_{Schnitt}$, für den $\dot{V}_{Schnitt} \geq 10\,l$/min und vorzugweise $\dot{V}_{Schnitt} \geq 60\,l$/min gilt. Die Dichte des Atemgases wird bestimmt durch die Zusammensetzung aus Luft, Sauerstoff, Lachgas und volatilen Anästhetika im Gasgemisch, wobei eine Atemgastemperatur von 20°C zugrunde gelegt wird.

Somit kann mit Hilfe von Gleichung (1) der Ventilkörper, auch wenn das Narkosegas und damit dessen Dichte nicht genau festgelegt sind, ohne Weiteres bestimmt werden.

Das zuvor beschriebene Beatmungssystem arbeitet wie folgt. In der Einatemphase ist der in der Ventilations- und Gasdosiereinrichtung 9 vorgesehene Beatmungsantrieb eingeschaltet, sodass sich das erste Rückschlagventil 7 öffnet und das PEEP-Ventil 15 ist geschlossen. Dadurch gelangt Atemgas, dessen Konzentration durch die Ventilations- und Gasdosiereinrichtung 9 gesteuert wird, über das Y-Stück 1 und die Anschlussleitung 3 zu dem Patienten, wobei ein Teil des Atemgases aus dem Handbeatmungsbeutel 29 kommt.

Beim Ausatmen ist das erste Rückschlagventil 7 aufgrund des treibenden Druck $\Delta P$, der vom Patienten erzeugt wird, geschlossen und das zweite Rückschlagventil 13 geöffnet, so dass das ausgeatmete Gas entlang des exspiratorischen Zweigs 11 durch das PEEP-Ventil 15 zu der Verzweigung 19 strömt, wobei das PEEP-Ventil 15 sicherstellt, dass der beispielsweise von der zweiten Messeinrichtung 12 erfasste Druck nicht unter eine vorgegebene Schwelle fällt.

Von der Verzweigung 19 strömt das Gas solange entlang der zweiten Zuleitung 27 in den Handbeatmungsbeutel 29, bis sich im exspiratorischen Zweig 11 ein Druck aufbaut, der im Vergleich zu dem Umgebungsdruck über dem Schwellendruck $\Delta P_{Schwelle}$ des NGF-Ventils 23 liegt. Erst dann kann das NGF-Ventil 23 öffnen, wobei dies nicht bereits vorher durch kurzzeitige Druckschwankungen bzw. Druckgradienten im Bereich von $\dfrac{\Delta p}{\Delta t} = \dfrac{5mbar}{0,5s}$ passieren kann, da das Öffnungsverhalten des NGF-Ventils 23 aufgrund der vergleichweise großen und gemäß Gleichung (1) festgelegten Masse $m_{Ventil}$ hinreichend träge ist.

In der nächsten Einatemphase strömt wieder Gas aus dem Handbeatmungsbeutel 29 durch den $CO_2$-Absorber 17 zurück in den inspiratorischen Zweig 5 und wird ggfs. in seiner Zusammensetzung durch die Ventilations- und Gasdosiereinrichtung 9 verändert und dann dem Patienten über das Y-Stück 1 zugeführt.

Wie aus dem Vorstehenden hervorgeht, ist das erfindungsgemäße Beatmungssystem durch das NGF-Ventil 23, dessen Ventilkörper 37 entsprechend den Gegebenheiten in dem System dimensioniert ist, derart ausgestaltet, dass das NGF-Ventil 23 nur dann öffnet, wenn sich im exspiratorischen Zweig für einen längeren Zeitraum ein Druck aufbaut, dessen Differenz zu dem Umgebungsdruck über dem Schwellendruck $\Delta P_{Schwelle}$ liegt, sodass der Verlust an Narkosegas minimiert wird.

**Patentansprüche**

1. Beatmungssystem zum Zuführen eines Narkosegases mit einer Dichte $\rho_{Gas}$,
   mit einem Y-Stück (1), das zur Verbindung mit einem Patienten vorgesehen ist,
   mit einem Beatmungskreis, in dem ein inspiratorischer Zweig (5) und ein exspiratorischer Zweig (11) vorgesehen ist, die sich von dem Y-Stück (1) weg erstrecken,
   wobei in dem exspiratorischen Zweig (11) eine Verzweigung (19) vorgesehen ist,
   wobei von der Verzweigung (19) eine erste Zuleitung (21) zu einem Narkosegasfortleitungsventil (23) führt,
   wobei von der Verzweigung (19) eine zweite Zuleitung (27) zu einem Reservoir (29) führt, die eine Querschnittsfläche $A_{Zuleitung2}$ und eine Länge $l_{Zuleitung2}$ aufweist,
   wobei das Narkosegasfortleitungsventil (23) einen Ventilkörper (37) mit einer Masse $m_{Ventil}$ aufweist,
   **dadurch gekennzeichnet,**
   **dass** der Ventilkörper (37) durch Schwerkraftwirkung hin zu der ersten Zuleitung (21) gegen einen kreisringförmigen Ventilsitz (33) mit einem Durchmesser $d_{Ventil}$ gedrückt wird und
   **dass** ein Vorspannmittel (41) vorgesehen ist, das auf den Ventilkörper (37) eine Vorspannkraft entgegen der Wirkung der Schwerkraft (G) ausübt, sodass die Masse $m_{Ventil}$ und die Vorspannkraft einen Schwellendruck $\Delta P_{Schwelle}$ festlegen, um den der Druck in der ersten Zuleitung (21) über dem auf der der ersten Zuleitung (21) abgewandten Seite des Narkosegasfortleitungsventils (23) mindestens liegen muss, damit dieses öffnet.

2. Beatmungssystem nach Anspruch 1, wobei die Masse $m_{ventil}$ des Ventilkörpers (37) derart gewählt ist, dass

$$m_{Ventil} > \frac{\sqrt{2} \cdot \pi^2 \cdot l_{Zuleitung2}^2 \cdot \dot{V}_{Schnitt} \cdot \rho_{Gas}^2 \cdot d_{Ventil}^3 \cdot \left(\Delta P - \Delta P_{Schwelle}\right)}{8 \cdot A_{Zuleitung2}^2 \cdot \Delta P^2 \cdot \sqrt{\dfrac{\rho_{Gas}}{\Delta P}}}$$

gilt, wobei $\dot{V}_{Schnitt} \geq 10 l/min$, $\Delta P = 5mbar$ und $\Delta P_{Schwelle} = 1,1\ mbar$ ist und die Dichte $\rho_{Gas}$ zwischen 1, 12 kg/m$^3$ und 2,19 kg/m$^3$ liegt.

3. Beatmungssystem nach Anspruch 2, wobei $\dot{V}_{Schnitt} \geq 60 l/min$ gilt.

4. Beatmungssystem nach einem der Ansprüche 1 bis 3, wobei das Reservoir als ein flexibler Handbeatmungsbeutel (29) mit einem variablen Volumen ausgebildet ist.

5. Beatmungssystem nach einem der Ansprüche 1 bis 4, wobei das Narkosegasfortleitungsventil (23) ein Ventilgehäuse (31) aufweist, in dem der Ventilsitz (33) vorgesehen ist, wobei der Ventilsitz (33) in einer senkrecht zur Richtung der Schwerkraft verlaufenden Sitzebene (35) verläuft und in eine der Richtung (G) der Schwerkraft entgegengesetzte Richtung weist und wobei der Ventilkörper (37) derart angeordnet ist, dass er unter Wirkung der Schwerkraft auf dem Ventilsitz (33) aufliegt.

6. Beatmungssystem nach Anspruch 4, wobei als Vorspannmittel eine Schraubenfeder (41) vorgesehen ist, die mit einem ersten Ende (43) an der dem Ventilkörper (37) anliegt, die entgegen der Wirkung der Schwerkraft auf den Ventilkörper (37) drückt und die auf der in Richtung (G) der Schwerkraft weisenden Seite des Ventilkörpers (37) angeordnet ist.

7. Beatmungssystem nach Anspruch 6, wobei eine Stellschraube (39) im Ventilgehäuse (31) axial verstellbar gehaltert ist und wobei sich das zweite Ende (45) der Schraubenfeder (41) an der Stellschraube (39) abstützt.

**Claims**

1. Respiration system for supplying an anaesthetic gas having a density $\rho_{gas}$, with a Y-piece (1) which has been provided for connection to a patient, with a respiration circuit in which an inspiratory branch (5) and an expiratory branch (11) have been provided, which extend away from the Y-piece (1), wherein a branching (19) has been provided in the expiratory branch (11), wherein a first feeder (21) leads from the branching (19) to an anaesthetic-gas conduction valve (23), wherein a second feeder (27) leads from the branching (19) to a reservoir (29), said second feeder having a cross-sectional area $A_{feeder2}$ and a length $l_{feeder2}$, wherein the anaesthetic-gas conduction valve (23) has a valve body (37) having a mass $m_{valve}$, **characterised in that** the valve body (37) is pressed by gravitational action towards the first feeder (21) against an annular valve seat (33) having a diameter $d_{valve}$ and **in that** a biasing means (41) has been provided which exerts a biasing force on the valve body (37) contrary to the action of gravity (G), so that the mass $m_{valve}$ and the biasing force define a threshold pressure $\Delta P_{threshold}$ by which the pressure in the first feeder (21) must at least lie above that on the side of the anaesthetic-gas conduction valve (23) facing away from the first feeder (21), in order that said valve opens.

2. Respiration system according to Claim 1, wherein the mass $m_{valve}$ of the valve body (37) has been chosen in such a way that

$$m_{valve} > \frac{\sqrt{2} \cdot \pi^2 \cdot l_{feeder2}^2 \cdot \dot{V}_{mean} \cdot \rho_{gas}^2 \cdot d_{valve}^3 \cdot \left(\Delta P - \Delta P_{threshold}\right)}{8 \cdot A_{feeder2}^2 \cdot \Delta P^2 \cdot \sqrt{\dfrac{\rho_{gas}}{\Delta P}}}$$

holds, wherein $\dot{V}_{mean} \geq 10 l/min$, $\Delta P = 5$ mbar and $\Delta P_{threshold} = 1.1$ mbar, and the density $\rho_{gas}$ lies between 1.12 kg/m$^3$ and 2.19 kg/m$^3$.

3. Respiration system according to Claim 2, wherein $\dot{V}_{mean} \geq 60 l/min$ holds.

4. Respiration system according to one of Claims 1 to 3, wherein the reservoir takes the form of a flexible manual respiration bag (29) having a variable volume.

5. Respiration system according to one of Claims 1 to 4, wherein the anaesthetic-gas conduction valve (23) has a valve casing (31) in which the valve seat (33) has been provided,
wherein the valve seat (33) extends in a seat plane (35) extending perpendicular to the direction of gravity, and points in a direction opposite to the direction (G) of gravity and
wherein the valve body (37) has been arranged in such a way that it bears against the valve seat (33) under the action of gravity.

6. Respiration system according to Claim 4, wherein by way of biasing means a helical spring (41) has been provided, which with a first end (43) bears against the valve body (37), which presses on the valve body (37) contrary to the action of gravity, and which has been arranged on the side of the valve body (37) pointing in the direction (G) of gravity.

7. Respiration system according to Claim 6, wherein a set screw (39) has been retained in the valve casing (31) so as to be axially adjustable and
wherein the second end (45) of the helical spring (41) is supported against the set screw (39).

## Revendications

1. Système de ventilation pour amener un gaz anesthésique avec une masse volumique $\rho_{Gas}$,
comprenant un raccord en Y (1) qui est prévu pour être relié à un patient,
comprenant un circuit de ventilation dans lequel sont prévues une branche inspiratoire (5) et une branche expiratoire (11) qui s'étendent à partir du raccord en Y (1),
un branchement (19) étant prévu dans la branche expiratoire (11),
une première conduite d'amenée (21) menant du branchement (19) à une valve d'évacuation de gaz anesthésique (23),
une deuxième conduite d'amenée (27) menant du branchement (19) à un réservoir (29) et présentant une aire de section transversale $A_{Zuleitung2}$ et une longueur $l_{Zuleitung2}$,
la valve d'évacuation de gaz anesthésique (23) présentant un corps de valve (37) avec une masse $m_{Ventil}$,
**caractérisé en ce que** le corps de valve (37) est poussé sous l'effet de la gravité en direction de la première conduite d'amenée (21), contre un siège de valve (33) circulaire de diamètre $d_{Ventil}$, et
**en ce qu'**il est prévu un moyen de précontrainte (41) qui exerce une force de précontrainte sur le corps de valve (37), à l'encontre de l'action de la gravité (G), de sorte que la masse $m_{Ventil}$ et la force de précontrainte définissent une pression de seuil $\Delta P_{Shwelle}$ de la valeur de laquelle la pression dans la première conduite d'amenée (21) doit au moins être supérieure à celle sur le côté de la valve d'évacuation de gaz anesthésique (23) qui est opposé à la première conduite d'amenée (21), afin que ladite valve s'ouvre.

2. Système de ventilation selon la revendication 1, dans lequel la masse $m_{Ventil}$ du corps de valve (37) est choisie telle que

$$m_{Ventil} > \frac{\sqrt{2} \cdot \pi^2 \cdot l_{Zuleitung2}^2 \cdot \dot{V}_{Schnitt} \cdot \rho_{Gas}^2 \cdot d_{Ventil}^3 \cdot \left(\Delta P - \Delta P_{Schwelle}\right)}{8 \cdot A_{Zuleitung2}^2 \cdot \Delta P^2 \cdot \sqrt{\dfrac{\rho_{Gas}}{\Delta P}}}$$

avec $\dot{V}_{Schnitt} \geq 10\,l/min$, $\Delta P = 5$ mbars et $\Delta P_{Schwelle} = 1{,}1$ mbars, et la masse volumique $\rho_{Gas}$ étant comprise entre 1,12 kg/m$^3$ et 2,19 kg/m$^3$.

3. Système de ventilation selon la revendication 2, dans lequel $\dot{V}_{Schnitt} \geq 60\,l/min$.

4. Système de ventilation selon l'une des revendications 1 à 3, dans lequel le réservoir est réalisé sous la forme d'une poche de ventilation manuelle (29) souple à volume variable.

5. Système de ventilation selon l'une des revendications 1 à 4, dans lequel la valve d'évacuation de gaz anesthésique (23) présente une cage de valve (31) dans laquelle est prévu le siège de valve (33),
le siège de valve (33) s'étendant dans un plan de siège (35) qui est perpendiculaire au sens de la gravité, et étant orienté dans un sens qui est opposé au sens (G) de la gravité, et
le corps de valve (37) étant disposé de telle manière qu'il soit en appui sur le siège de valve (33) sous l'effet de la gravité.

6. Système de ventilation selon la revendication 4, dans lequel il est prévu, en tant que moyen de précontrainte, un ressort hélicoïdal (41) qui est en appui par une première extrémité (43) contre le corps de valve (37), qui pousse le corps de valve (37) à l'encontre de l'effet de la gravité et qui est disposé sur le côté du corps de valve (37) orienté dans le sens (G) de la gravité.

7. Système de ventilation selon la revendication 6, dans lequel une vis de réglage (39) est montée avec possibilité de déplacement axial dans la cage de valve (31), et
dans lequel la deuxième extrémité (45) du ressort hélicoïdal (41) prend appui sur la vis de réglage (39).

Fig. 1

**Fig.2**

**EP 2 540 334 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0894506 B1
- DE 102004052398 B3
- US 4207884 A
- DE 10041007 C1